# EUROPEAN PATENT APPLICATION

(11) **EP 2 380 619 A1**
(43) Date of publication of application: **26.10.2011**
(21) Application number: 10160674.7
(22) Date of filing: 22.04.2010
(51) Int. Cl.: A61M 21/00

(54) **Illumination system for influencing the mental state of a person**

(71) Applicant: Philips Intellectual Property & Standards GmbH, 20099 Hamburg (DE); Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The invention relates to an illumination system for changing the brain state of a person (especially during sleep) by changing the color of the light emitted towards the eyes of said person.

## Description

### FIELD OF THE INVENTION

The present invention is directed to novel illumination systems, especially illumination systems for influencing and/or changing the mental and/or physical state of a person

### BACKGROUND OF THE INVENTION

Systems for changing the mental state of a person are known in the field, e.g. from the US 5,495,853 which uses photic stimulation delivered through the eyes in order to alter the brain state of a person.

However, there is still the continuing need for illumination systems which are able to influence and/or change the mental and/or physical state of a person, especially the sleeping state of a person.

### SUMMARY OF THE INVENTION

It is an object of the present invention to provide an illumination system, which is capable to influence and/or change the mental and/or physical state of a person, especially the sleeping state of a person.

This object is solved by an illumination system according to claim 1 of the present invention. Accordingly, an illumination system is provided comprising a light emitting system which when in use emits light towards the eyes of a person and a physical state influencing system which changes the color of the light emitted by the light emitting system to alter, influence and/or change the mental and/or physical state of a person.

Surprisingly the inventors of the present invention have found out that it is possible to alter, influence and/or change the mental and/or physical state of a person, especially the sleeping state of a person simply by changing the color of light emitted towards the eyes of said person.

It should be noted that the term "light emitting system" is to be understood in its broadest sense and includes LEDs, also arrays of LEDs, tube and discharge lamps, halogen lamps, excimer lamps and any combination of these elements. The light emitting system may include a system where several light emitters (such as LEDs) are provided in one "housing"; however, also other systems are included where large arrays or a multitude of housings are present.

It should be noted that "towards the eyes" is not limited to a physical state of said person where the eyes are open. As will be discussed and described later, the invention also deals with influencing the sleep of said person.

The term "changing the color of light" is to be understood in its broadest sense and especially includes (but is not limited to):
- Using "white light" but changing the color temperature and/or the color indices so that even after the change the light may seem still white, however, the x and y indices (on the CIE color triangle) have been altered
- Changing the light, e.g. from green to red

It should be noted that the terrm "changing the color of light" is not intended to mean and/or include that the illumination system works by changing this color only. The illumination system may also be adapted in order to change other parameters and/or features, such as (but not limited to) the intensity of the light, further IR and/or UV radiation, odors, sound, music and/or visual impressions.

Such an illumination has shown for a wide range of applications within the present invention to have at least one of the following advantages:
- The illumination system is much softer and requires less physical intervention than systems known in the art
- The illumination system requires no medication for the patient, thereby having no or almost no negative side effects

According to a preferred embodiment of the present invention, said mental and/or physical state comprises the sleeping state of said person.

The term "mental state" is to be understood in its broadest sense, for example well-balanced, nerveless, active, concentrated,... and especially means and/or includes sleep states, like sleep stadium 1 to 4 (4= deep sleep phase) or REM sleep phase and/or sleep-wake cycle and/or time to fall asleep.

According to a preferred embodiment of the present invention, said physical state influencing system changes the mental and/or physical state of said person by changing the color of the light emitted by the light emitting system according to a preselected pattern.

Said preselected pattern may be pre-set in said illumination system; however according to an alternative embodiment of the present invention, the illumination system may include a feedback system in order to allow the person using the illumination system to control the pattern.

According to a yet alternative embodiment of the present invention, the preselected pattern may be measured and/or determined individually by using EEG or other measurement methods. This will be described in greater detail later on.

It goes without saying that the illumination system may have more than one preselected pattern, either for the person to choose or individually set for different persons which want to use the illumination system.

It further goes without saying that the illumination system may have more than one preselected pattern also for the reason that different mental and/or physical state(s) want to be achieved. This will be described in greater detail later on.

According to a preferred embodiment, said pattern comprises changing the color of the light by changing the light within an area in the CIE color triangle, whereby said area is defined by including the area of all circles which centre lies at the edge of the color triangle and which radiae are ≧0.025 to ≤0.06.

This has been shown in practice to be one effectful way to alter the mental and/or physical state of most persons using the illumination system, especially the sleeping state. By "moving" the color of the light within this area it has been shown that for most persons the ratio of α-waves to β-waves in an awake state and the deep sleep phase to the REM phase during the sleeping state may be changed, thus either causing the person to sleep more relaxed and deep or - if moving "backwards" - causing the person to wake up more easier.

According to a preferred embodiment, said pattern comprises changing the color of the light by changing the light within an area in the CIE color triangle, whereby said area is defined by including the area of all circles which centre lies at the edge of the color triangle and which radiae are ≥0.03 to ≤ 0.05.

Such a change can according to one embodiment of the present invention be achieved by providing several (e.g. from four to nine) light sources such as LEDs with the light emitting system whose colors are within said area (preferably to somewhat "span") the area and then switching the light sources on and off.

According to a preferred embodiment, said pattern comprises changing the color of the light by changing the light within an area in the CIE color triangle, whereby said area is defined by including the area of all circles which centre lies on the black body line with a color temperature of ≥3000K to ≤10000K which radiae are ≥0.045 to ≤0.08.

This has been shown in practice to be another effectful way to alter the mental and/or physical state of most persons using the illumination system, especially the sleeping state. By "moving" the color of the light within this area it has been shown that for most persons the ratio of α-waves to β-waves in an awake state and the deep sleep phase to the REM phase during the sleeping state may be changed, thus either causing the person to sleep more relaxed and deep or - if moving "backwards" - causing the person to wake up more easier.

According to a preferred embodiment, said pattern comprises changing the color of the light by changing the light within an area in the CIE color triangle, whereby said area is defined by including the area of all circles which centre lies on the black body line with a color temperature of ≥3000K to ≤10000K, preferably ≥4000K to ≤9000K which radiae are ≥0.05 to ≤ 0.07.

Such a change can according to one embodiment of the present invention be achieved by providing several (e.g. from four to nine) light sources such as LEDs with the light emitting system whose colors are within said area (preferably to somewhat "span") the area and then choosing different combination of said light sources. Usually at least two light sources will be switched on at the same time.

The present invention furthermore relates to a method of altering, influencing and/or changing the mental and/or physical state of a person by changing the color of light emitted towards the eyes of said person.

According to a preferred embodiment, said mental and/or physical state comprises the sleeping state of said person.

According to a preferred embodiment, the method changes the color of light emitted towards the eyes of said person in order to influence the ratio of α-waves to β-waves in an awake state and the deep sleep phase to the REM phase during the sleeping state of said person,

The different waves (α,β,δ, REM-waves) may be measured using EEG or other brain observation methods. To this end, it is one embodiment of the present invention that the person who uses the illumination system (or test persons) are subjected to EEG or other brain observation methods while the illumination system is in use and color of light emitted towards the eyes of either said person or test persons is changed. Then the reaction of the brain (e.g. in terms of the α, β, δ-waves, their ratio or other suitable features) is measured and finally it is determined which "path" along the CIE color triangle is most suitabe either for this person or for the test persons. This data is then stored and used by the illumination system later on.

According to a preferred embodiment, said method comprises
a) Selecting a path along the CIE color triangly where the mental and/or physical state of said person can be altered, influenced and/or changed effectively
b) Chainging the mental and/or physical state of a person by changing the color of light emitted towards the eyes of said person along said path.

According to a preferred embodiment, said method comprises changing the color of the light by changing the light within an area in the CIE color triangle, whereby said area is defined by including the area of all circles which centre lies at the edge of the color triangle and which radiae are >0.025 to ≤0.06

According to a preferred embodiment, said method comprises comprises changing the color of the light by changing the light within an area in the CIE color triangle, whereby said area is defined by including the area of all circles which centre lies on the black body line with a color temperature of ≥3000K to ≤10000K which radiae are ≥0.045 to ≤0.08.

A Illumination system and/or a method according to the present invention may be of use in a broad variety of systems and/or applications, amongst them one or more of the following:
- Home lighting systems
- Professional lighting systems e.g. in hospitals
- Lighting systems in wellness farms

It should be noted that this invention and its use is not intended to illegitimate limit a medical practioner (such a physician, a surgeon or other qualified persons depending on local jurisdiction). Therefore therapeutic, diagnosic and/or chirurgical use of this invention by such practioners is explicitly disclaimed wherever jurisdiction demands.

The aforementioned components, as well as the claimed components and the components to be used in accordance with the invention in the described embodiments, are not subject to any special exceptions with respect to their size, shape, Illumination system selection and technical concept such that the selection criteria known in the pertinent field can be applied without limitations.

### BRIEF DESCRIPTION OF THE DRAWINGS

Additional details, features, characteristics and advantages of the object of the invention are disclosed in the subclaims, the figures and the following description of the respective figures and examples, which --in an exemplary fashion-- show several embodiments and examples of Illumination systems according to the invention.
Fig. 1 shows an image of the CIE color triangle with a set of LEDs within an area defined by all circles which centre lies at the edge of the color triangle and which radiae are 0.6
Fig. 2 shows the image of Fig. 1 with some of the circles
Fig. 3 shows an image of the CIE color triangle with a set of LEDs within an area defined by all circles which centre lies at the edge of the color triangle and which radiae are 0.4
Fig. 4 shows the image of Fig. 3 with some of the circles

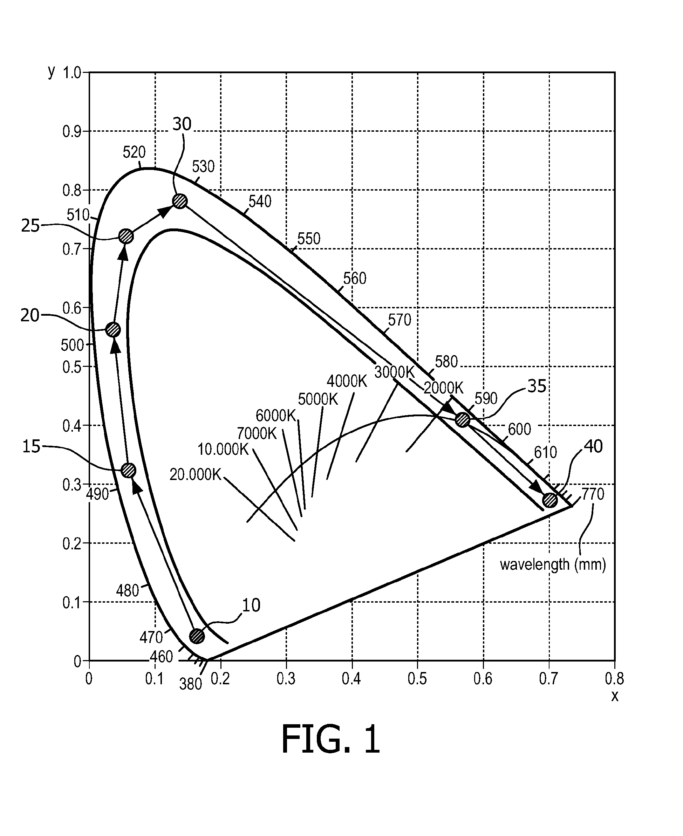

Fig. 1 shows an image of the CIE color triangle with a set of LEDs within an area defined by all circles which centre lies at the edge of the color triangle and which radiae are 0.6 (indicated by the two black lines). Altogether there are seven LEDs (10,15,20,25,30,35,40) which have color points to somewhat "span" the area. By "going" from one LED to another, e.g. by switching the LEDs one and off, it is possible to go from one end of the area (e.g. the left lower are) to the other (the right lower area) - as indicated by the arrows - thus influencing the mental state of the user.

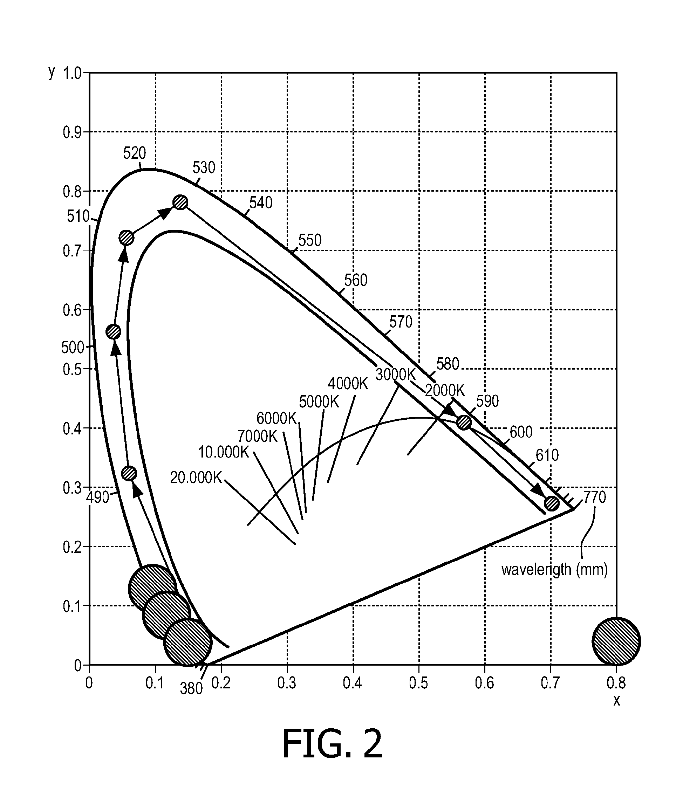

Fig. 2 shows the image of Fig. 1 with some of the circles (in the left lower corner) which have been used to "define" the area.

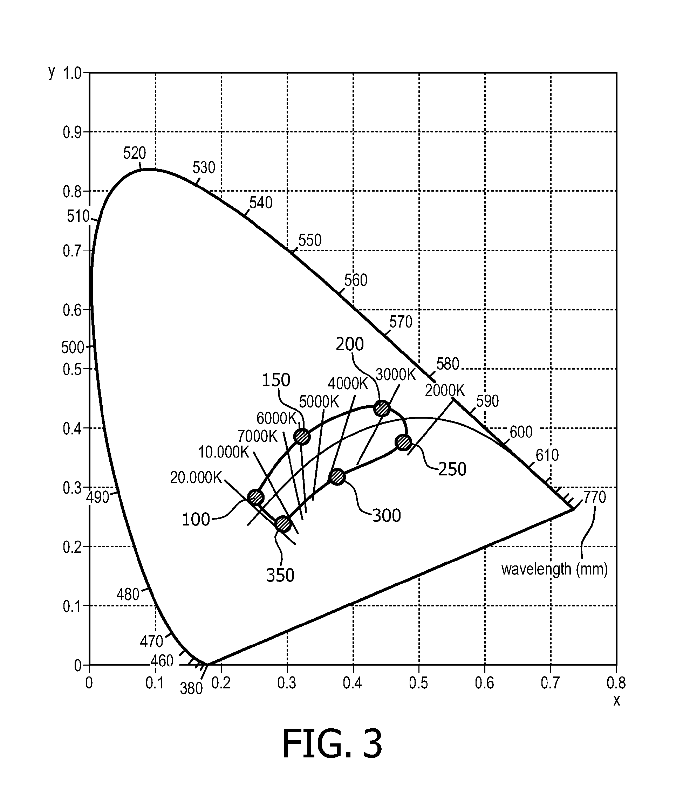

Fig. 3 shows an image of the CIE color triangle with a set of LEDs within an area defined by all circles which centre lies at the edge of the color triangle and which radiae are 0.4. Altogether there are six LEDs (100,150,200,250,300,350) which have color points to somewhat "span" the area. In this embodiment the color change occurs by switching from one "set" of LEDs to the other. E.g. the color could be changed by switching from LEDs 100 and 350 to LEDs 150 and 300 in that way that initially the LEDs 100 and 350 are turned on and then these are switched off and LEDs 150 and 300 are turned on. By doing so the overall color of the illumination system will still seem white although the color has been changed significantly. Of course other combinations or sets of LEDs may be used, too.

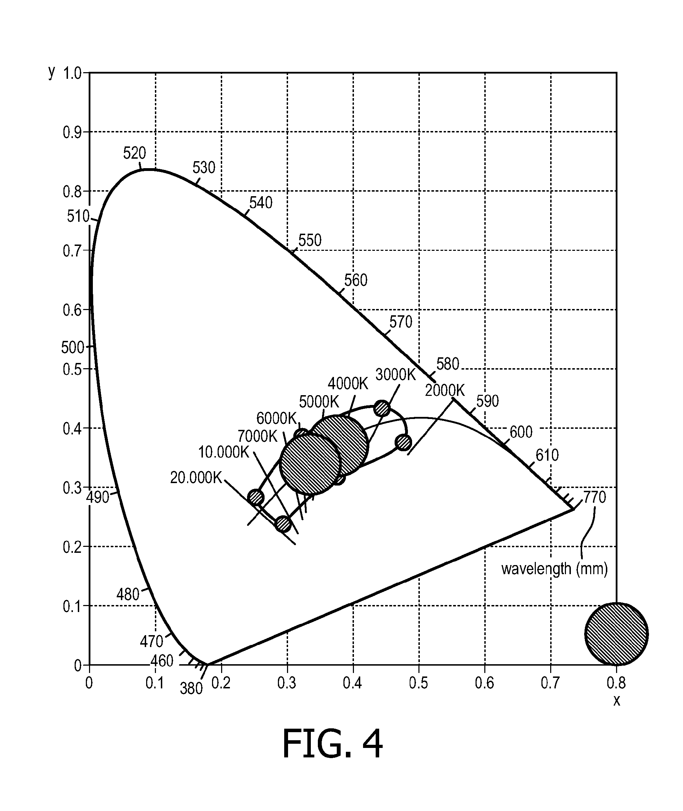

Fig. 4 shows the image of Fig. 3 with some of the circles (in the left lower corner) which have been used to "define" the area.

The invention will furthermore be understood by the following Inventive Example which is merely for illustration of the invention only and non- limiting.

### INVENTIVE EXAMPLE

### Part I: Selection of a suitable test person

Five volunteers were subjected to a test study. These volunteers specified that they would spend their morning (especially their breakfast) at a white light of 3000K (light bulbs) and approx. 300 Ix.

In a first study these volunteers were subjected to white light of 3000K and approx. 3000 Ix (i.e. ten times more intensity). After five days four of said five test person declared that they feel "more fresh more fit and more productive".

Further test studies were then only conducted with the only person who felt no difference, instead he declared the white light as being "somewhat unpleasent".

### Part II: Use of the illumination system according to the present invention

The only left volunteer test person then used an illumination system according to one embodiment of the present invention. In this illumination system, the physical state influencing system was adapted/programmed as to deliver first white light of 3000 K and 3000 Ix for 10 minutes, then 3500K and 3000 Ix (10 Minutes) and 4000K and 3000 Ix (10 Minutes), in accordance with the embodiments of Figs. 3 and 4.

As a result, said volunteer test person declared - only after one treatment - himself as being "much more fresh more fit and more productive", further said white light was no longer unpleasent to him.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. Illumination system comprising a light emitting system which when in use emits light towards the eyes of a person and a physical state influencing system which changes the color of the light emitted by the light emitting system to alter, influence and/or change the mental and/or physical state of said person.

2. The Illumination system of Claim 1, whereby said mental and/or physical state comprises the sleeping state of said person.

3. The Illumination system of Claim 1 or 2, whereby said physical state influencing system changes the mental and/or physical state of said person by changing the color of the light emitted by the light emitting system according to a preselected pattern

4. The Illumination system of claim 3, whereby said pattern comprises changing the color of the light by changing the light within an area in the CIE color triangle, whereby said area is defined by including the area of all circles which centre lies at the edge of the color triangle and which radiae are ≥0.025 to ≤0.06.

5. The Illumination system of claim 3 whereby said pattern comprises changing the color of the light by changing the light within an area in the CIE color triangle, whereby said area is defined by including the area of all circles which centre lies on the black body line with a color temperature of ≥3000K to ≤10000K which radiae are ≥0.045 to ≤0.08.

6. A method of altering, influencing and/or changing the mental and/or physical state of a person by changing the color of light emitted towards the eyes of said person.

7. The method of claim 6, whereby said mental and/or physical state comprises the sleeping state of said person

8. The method of claim 7, whereby said method changes the color of light emitted towards the eyes of said person in order to influence the a-waves during the sleeping state of said person

9. The method of any of the claims 6 to 8, comprising
a) Selecting a path along the CIE color triangly where the mental and/or physical state of said person can be altered, influenced and/or changed effectively
b) Chainging the mental and/or physical state of a person by changing the color of light emitted towards the eyes of said person along said path.

10. A system comprising a Illumination system according to any of the claims 1 to 5 and/or using the method of any of the claims 6 to 9, the system being used in one or more of the following applications:
- Home lighting systems
- Professional lighting systems e.g. in hospitals
- Lighting systems in wellness farms
